(19) Europäisches Patentamt
European Patent Office
Office européen des brevets

(11) Veröffentlichungsnummer: **0 458 214 A1**

# (12) EUROPÄISCHE PATENTANMELDUNG

(21) Anmeldenummer: **91108054.7**

(22) Anmeldetag: **17.05.91**

(51) Int. Cl.$^5$: **C07D 453/02**, C07D 471/08, C07D 487/08, A61K 31/435, A61K 31/55, A61K 31/395

(30) Priorität: **19.05.90 DE 4016212**
**13.06.90 DE 4018886**

(43) Veröffentlichungstag der Anmeldung:
**27.11.91 Patentblatt 91/48**

(84) Benannte Vertragsstaaten:
**AT BE CH DE DK ES FR GB GR IT LI LU NL SE**

(71) Anmelder: **BOEHRINGER INGELHEIM KG**

**W-6507 Ingelheim am Rhein(DE)**
(84) **BE CH DE DK ES FR GR IT LI LU NL SE AT**

Anmelder: **BOEHRINGER INGELHEIM INTERNATIONAL G.M.B.H.**

**W-6507 Ingelheim am Rhein(DE)**
(84) **GB**

(72) Erfinder: **Walther, Gerhard, Dr.**
**Pfarrer-Heberer-Strasse 37**
**W-6530 Bingen(DE)**
Erfinder: **Küfner-Mühl, Dr.**
**Schlossbergstrasse 8**
**W-6507 Ingelheim 4(DE)**
Erfinder: **Stransky, Werner, Dr.**
**Im Hippel 24**
**W-6535 Gau-Algesheim(DE)**
Erfinder: **Weber, Karl-Heinz, Dr.**
**Kaiser-Karl-Strasse 11**
**W-6535 Gau-Algesheim(DE)**
Erfinder: **Ensinger, Helmut, Dr.**
**Magdeburger Strasse 54**
**W-6507 Ingelheim am Rhein(DE)**
Erfinder: **Kuhn, Franz Josef, Dr.**
**Beethovenstrasse 11**
**W-6535 Gau-Algesheim(DE)**
Erfinder: **Müller, Enzio, Prof. Dr.**
**Grundstrasse 22**
**W-6507 Ingelheim am Rhein(DE)**

(54) **Bicyclische 1-Aza-cycloalkane.**

(57) Die Erfindung betrifft bicyclische 1-Aza-cycloalkane, Verfahren ihrer Herstellung sowie ihre Verwendung als Arzneimittel mit cholinometischen Eigenschaften.

EP 0 458 214 A1

Die Erfindung betrifft bicyclische 1-Aza-cycloalkane der allgemeinen Formel I, Verfahren zu ihrer Herstellung und ihre Verwendung als Arzneimittel.

Die neuen Verbindungen entsprechen der allgemeinen Formel

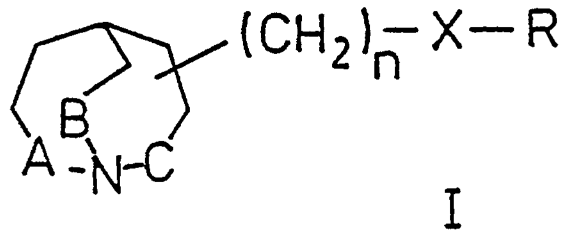

I

worin

| | |
|---|---|
| R | einen Alkylrest mit 1 bis 6 Kohlenstoffatomen, einen Alkenylrest mit 3 bis 6 Kohlenstoffatomen, einen Alkinylrest mit 3 bis 6 Kohlenstoffatomen, wobei der Alkyl-, Alkenyl oder Alkinylrest durch Phenyl, substituiertes Phenyl, gegebenenfalls substituiertes Biphenyl, ein gegebenenfalls substituiertes Oxetan oder einen gegebenenfalls substituierten 5-, 6- oder 7-gliedrigen Heterocyclus substituiert sein kann, oder |
| R | ein gegebenenfalls substituiertes Phenyl, gegebenenfalls substituiertes Biphenyl, einen gegebenenfalls substituierten 5-, 6- oder 7-gliedrigen Heterocyclus; |
| X | Sauerstoff oder Schwefel, |
| A, B und C | unabhängig voneinander $CH_2$ oder eine Einfachbindung und |
| n | = 0, 1 oder 2 |

bedeuten können, gegebenenfalls in Form ihrer Racemate, ihrer Enantiomere, ihrer Diastereomere und ihrer Gemische sowie gegebenenfalls ihre pharmakologisch unbedenkliche Säureadditionssalze wie auch ihre Quartärsalze.

Als Alkylgruppen im Sinne dieser Erfindung werden verzweigte oder unverzweigte Alkylreste mit 1 bis 6 Kohlenstoffatomen, wie z. B. Methyl, Ethyl, Propyl, Butyl, Pentyl und Hexyl wie auch deren verzweigte Isomere, wie z.B. iso-Propyl, iso-Butyl, tert.-Butyl, sec.-Butyl u.a., verstanden; unter Alkenylgruppen werden verzweigte oder unverzweigte Alkenylreste mit 3 bis 6 Kohlenstoffatomen - wie z.B. Propenyl, Butenyl, Pentenyl und Hexenyl - mit einer Doppelbindung; und unter Alkinylgruppen werden verzweigte oder unverzweigte Alkinylreste mit 3 bis 6 Kohlenstoffatomen - wie z.B. Propinyl, Butinyl und Pentinyl - mit einer Dreifachbindung verstanden. Bevorzugt sind solche Alkenyl- oder Alkinylreste, in denen die Doppel- bzw. Dreifachbindung endständig angeordnet ist.

Als substituiertes Phenyl werden - soweit nicht anders bezeichnet - Phenylreste verstanden, die ein-, zwei- oder dreifach durch Halogen, Hydroxy- und/oder verzweigtes oder unverzweigtes Alkyl mit 1 bis 5 Kohlenstoffatomen, $C_3$ - $C_6$-Cycloalkyl, $C_1$-$C_5$-Hydroxyalkyl, Amino, mono- oder disubstituiertes $C_1$ - $C_4$-Alkylamino substituiert sind. Gegebenenfalls substituiertes Cycloalkyl, Biphenyl, wobei der zweite Phenylring auch wie oben angegeben, substituiert sein kann.

Als Beispiele werden genannt:

2-Chlorphenyl, 2,6-Dichlorphenyl, 2-Bromphenyl, 3-Fluorphenyl, 2,3-Dichlorphenyl, 4-Hydroxyphenyl, 2-Methylphenyl, 4-Methylphenyl, 3-Ethylphenyl, 4-Propylphenyl, 4-Isopropylphenyl, 4-Butylphenyl, 4-tert.-Butylphenyl, 4-Pentylphenyl, 2,4-Dimethylphenyl, 2-Trifluoromethylphenyl, 3-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl,

Heterocyclus im Rahmen der oben angegebenen Definition steht unter anderem für einen 5- bis 7-gliedrigen Ring, der als Heteroatome Sauerstoff, Schwefel und/oder Stickstoff enthält und an den gegebenenfalls ein weiterer aromatischer Ring, bevorzugt ein Phenylring, ankondensiert sein kann.

Als Beispiele für gesättigte heterocyclische 6-gliedrige Ringe werden beispielsweise genannt:

1,4 und 1,3-Dioxan, Morpholin, Thiomorpholin, Piperidin, Piperazin, 4- $C_1$ bis $C_4$-Alkylpiperazin, N-Hydroxy - $C_1$ bis $C_4$ - Alkylpiperazin, 2,5-Diketopiperazin, Tetrahydropyran, Als Beispiele für gesättigte heterocyclische 5-gliedrige Ringe werden beispielsweise genannt:

Tetrahydropyrrol, Tetrahydrofuran, Prolin, Tetrahydropyrazol, Imidazolidin, Hydroxyprolin, Pyrrolidin, Pyrazolidin, Pyrrolidon, Thiolan, Butyrolacton, 1,2-Oxathiolan.

Als Beispiel für 5-, 6- und 7-gliedrige ein- und mehrfach ungesättigte heterocyclische Ringe werden beispielsweise genannt:

Imidazol, Imidazolin, 1,2,4- und 1,2,3-Triazol, Tetrazol, Isothiazol, Furan, Dihydrofuran, Thiophen, Pyridin, Pyrimidin, Pyran, 2,5-Dihydropyrrol, Thiazol, Thiadiazin, Azepin, 1,2-Oxathiepan, Pyrrol, Pyrrolin, Pyrazolin, Dimethylpyrrol, 2-Acylfuran, Dihydrothiophen. Als weitere Heteroarylreste seien beispielsweise Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.
Bevorzugter heterocyclischer Rest ist beispielsweise der Pyridinrest, dieser kann 1- bis 4-fach, bevorzugt 1- bis 2-fach gleich oder verschieden durch Hydroxy, $C_1$ - $C_4$-Alkyl, $C_1$ - $C_4$-Alkoxy, $NH_2$, mono oder di ($C_1$ - $C_4$-)Alkylamino, Halogen, $CF_3$,CN oder Nitro substituiert sein.

Als über ein Stickstoffatom gebundener 5, 6 oder 7-gliedriger heterocyclischer Ring werden außerdem beispielhaft genannt:
Phenantridin-6-on, Chinolin-2-on, Isochinolin-1,3-dion, Benz[c,d]indol, 1,4-Benzoxazin-3-on, Indol-2,3-dion, Indol-2-on, 1,2,4-Triazolo[4,3-a]pyridin-3-on, 1,2-Benzisothiazol-3-on, 1H-Indazol, 1H-Benzimidazol, 1H-Benztriazol, Benzothiazin-3-on, Benz[d,e]isochinolin-1,3-dion, 4-Chinazolinon, Isoindol-1-on, Pyrrolo[1,2-c]-imidazol-1,3-dion, 1,3-Dihydro-2H-indol-2-on, Tetrahydro-1H-isoindol-1,3-dion, 3,7-Dihydro-1H-Purin-2,6-dion, Indol, Indazol, Benzimidazol, Benzimidazol-2-on, 1,4-Benzothiazin-3-on-, 1H-Isoindol-1,3-dion.

Der Heterocyclus kann ein- oder mehrfach durch Halogen, Hydroxy, verzweigtes oder unverzweigtes $C_1$ bis $C_4$ Alkyl, $C_1$ bis $C_4$ Hydroxyalkyl und/oder $C_1$ bis $C_4$ Alkoxy substituiert sein. Der Heterocyclus kann eine Ketofunktion enthalten, wie z.B. Dihydrotetrafuranon.

Bevorzugte Verbindungen der allgemeinen Formel I sind solche, worin X Sauerstoff, n = 0 oder 1 und der Substituent $(CH_2)_n$-X-R in $\alpha$ oder $\beta$-Position zum carbocyclischen Brückenkopf steht, und R eine Alkylgruppe mit 1 bis 3 Kohlenstoffatomen, eine Alkenylgruppe mit 3 oder 4 Kolenstoffatomen, eine Alkinylgruppe mit 3 oder 4 Kohlenstoffatomen, eine gegebenenfalls substituierte Phenylgruppe, einen gegebenenfalls substituierten 5- oder 6-gliedrigen aromatischen Heterocyclus bedeutet.

Bevorzugte Verbindungen der allgemeinen Formel 1 sind Quinuclidine, worin n = 0 oder 1, X = Sauerstoff und R = ein gegebenenfalls substituiertes Pyridin, Thiophen, Furan, Pyrimidin, Imidazol, Pyrazol, 1,2,4-Triazol, Oxetan, Tetrahydrofuran, Pyrrolidin oder Oxolan, ein gegebenenfalls substituierter Phenylrest, ein gegebenenfalls substituierter Benzylrest ist, wobei die Seitenkette in der 3-Position des Quinuclidin-Systems angeordnet ist.

Bevorzugte 1-Azabicyclo[2.2.1]heptane der allgemeinen Formel I sind solche, worin X = Sauerstoff, n = 0 oder 1 und R eine Alkylgruppe mit 1 - 3 Kohlenstoffatomen, eine Alkenylgruppe mit 3 oder 4 Kohlenstoffatomen oder eine Alkinylgruppe mit 3 oder 4 Kohlenstoffatomen, ein gegebenenfalls substituiertes Pyridin, Thiophen, Furan, Pyrimidin, Imidazol, Pyrazol, 1,2,4-Triazol, Oxetan, Tetrahydrofuran, Pyrrolidin oder Oxolan, ein gegebenenfalls substituierter Phenylrest, ein gegebenenfalls substituierter Benzylrest, wobei die Seitenkette in der 3-Position des bicyclischen Systems angeordnet ist.

Bevorzugte 1-Aza-bicyclo[3.2.1]octane der allgemeinen Formel I sind solche, worin X = Sauerstoff, n = 0 oder 1 und R eine Alkylgruppe mit 1 - 3 Kohlenstoffatomen, eine Alkenylgruppe mit 3 oder 4 Kohlenstoffatomen oder eine Alkinylgruppe mit 3 oder 4 Kohlenstoffatomen, ein gegebenenfalls substituiertes Pyridin, Thiophen, Furan, Pyrimidin, Imidazol, Pyrazol, 1,2,4-Triazol, Oxetan, Tetrahydrofuran, Pyrrolidin oder Oxolan, ein gegebenenfalls substituierter Phenylrest, ein gegebenenfalls substituierter Benzylrest, wobei die Seitenkette in der 3- oder 6-Position des Bicyclus angeordnet ist.
Bevorzugte 1-Aza-bicyclo[3.3.1]nonane der allgemeinen Formel I sind solche, worin X = Sauerstoff, n = 0 oder 1 und R eine Alkylgruppe mit 1 bis 3 Kohlenstoffatomen, eine Alkenylgruppe mit 3 oder 4 Kohlenstoffatomen, eine Alkinylgruppe mit 3 oder 4 Kohlenstoffatomen, ein gegebenenfalls substituiertes Pyridin, Thiophen, Furan, Pyrimidin, Imidazol, Pyrazol, 1,2,4-Triazol, Oxetan, Tetrahydrofuran, Pyrrolidin oder Oxolan, ein gegebenenfalls substituierter Phenylrest, ein gegebenenfalls substituierter Benzylrest, wobei die Seitenkette bevorzugt in der 3-Position des Bicyclus angeordnet ist.

Besonders bevorzugt sind Verbindungen der allgemeinen Formel

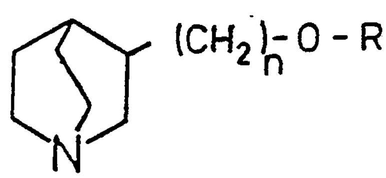

worin

n = 0 oder 1;

und

R einen Rest der Formel

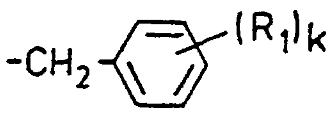

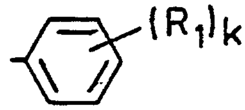

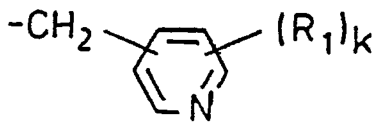

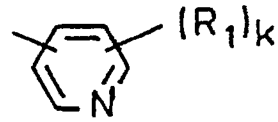

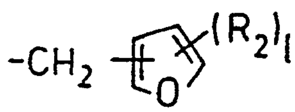

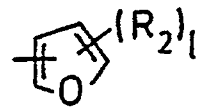

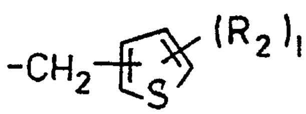

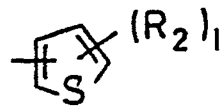

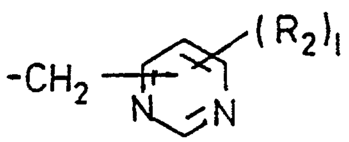

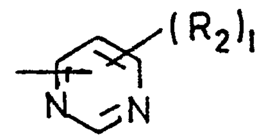

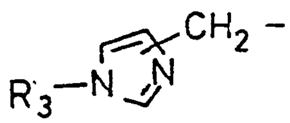

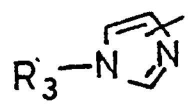

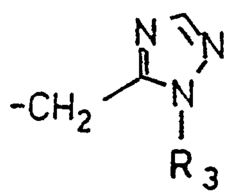

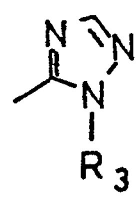

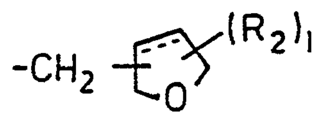

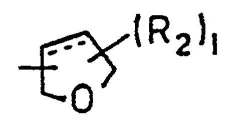

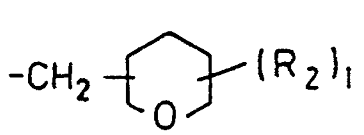

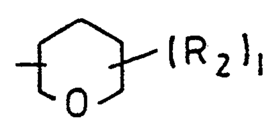

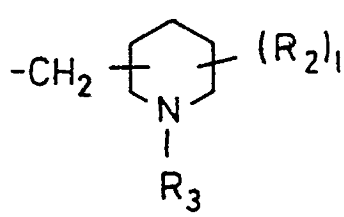

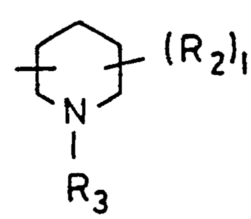

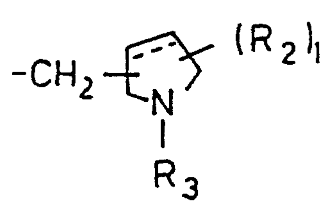

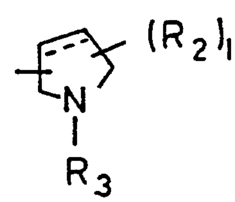

worin

$R_1$ Wasserstoff, $C_1$ - $C_4$ Alkyl, bevorzugt Methyl, $C_1$ - $C_4$-Alkoxy, bevorzugt Methoxy, Amino, $C_1$ - $C_4$-Alkylamino, $C_1$ - $C_4$-Dialkylamino, Halogen, Hydroxy, $C_3$ - $C_6$-Cycloalkyl, gegebenenfalls substituiertes Phenyl, = 0;

k 1, 2 oder 3, wobei k > 1 alle $R_1$ gleich oder verschieden sein können,

$R_2$ Wasserstoff, $C_1$ - $C_4$-Alkyl, bevorzugt Methyl, $C_1$-$C_4$-Alkoxy, bevorzugt Methoxy, Halogen, = 0;

l 1 oder 2, bevorzugt 1, wobei bei l = 2 $R_2$ gleich oder verschieden sein kann;

$R_3$ Wasserstoff, $C_1$-$C_4$-Alkyl, bevorzugt Methyl bedeuten können.

Bevorzugt sind ferner Reste der allgemeinen Formel

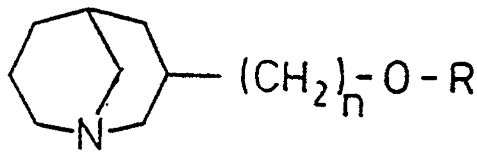

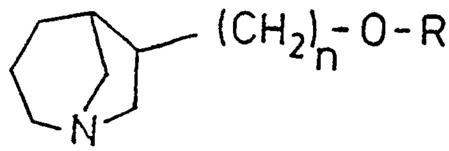

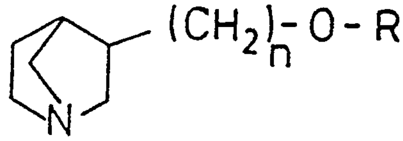

worin

n = null oder 1, bevorzugt null;

R = $C_3$-Alkinyl, $C_3$-Alkenyl, einen Rest der allgemeinen Formel

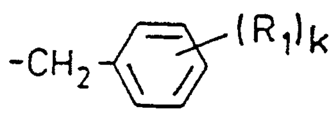

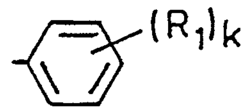

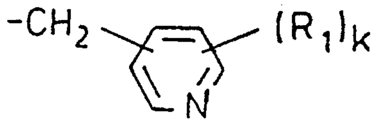

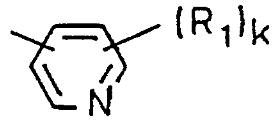

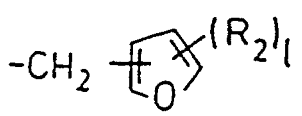

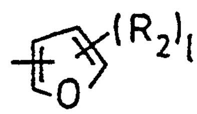

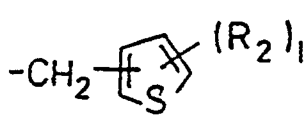

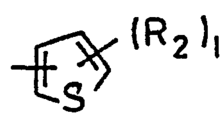

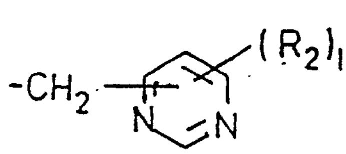

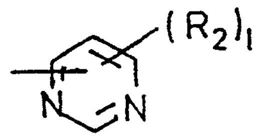

worin $R_1$, $R_2$, k und l wie zuvor definiert sind; bevorzugt sind ferner Reste der allgemeinen Formel

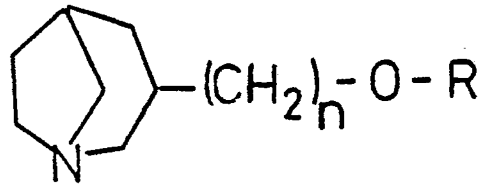

worin

n = null oder 1;

R = $C_3$-Alkinyl, $C_3$-Alkenyl, Methyl, Ethyl, Propyl,

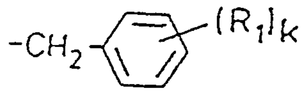

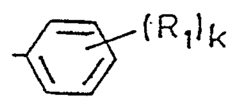

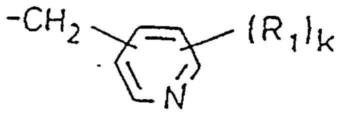

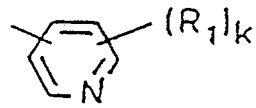

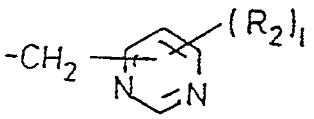

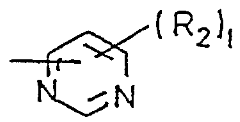

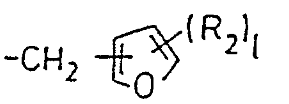

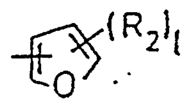

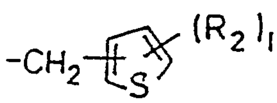

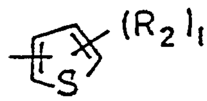

worin $R_1$, $R_2$, k und l wie zuvor definiert sind, in Form ihrer Racemate, ihrer Enantiomere, ihrer Diastereomere und ihrer Gemische sowie ihre pharmakologisch unbedenklichen Säureadditionssalze wie auch ihre Quartärsalze.

Aus dem Stand der Technik sind bestimmte Verbindungen der allgemeinen Formel I beschrieben. So zum Beispiel aus der nicht vorveröffentlichten Europäischen Patentanmeldung 370 415, aus der Quinuclidine der allgemeinen Formel I, worin R einen unsubstituierten Alkyl-, Alkenyl- oder Alkinylrest bedeutet, bekannt sind. Diese Verbindungen sind zu disclaimen.

In der Europäischen Patentanmeldung sind die optisch aktiven Verbindungen dieses Typs jedoch nicht namentlich genannt.

Die Trennung der Enantiomere erfolgt, wie im Beispiel 3 jetzt erstmals beschrieben, ausgehend von den racemischen Verbindungen unter Verwendung von optisch aktiven Salzen, z.B. (+) oder (-) Weinsäure, nach an sich bekannten Verfahren.

Anhand der Bindungsstudien an den Muskarinsubtypen $M_1$, $M_2$ und $M_3$ (Ratte) und anhand der Stimulation des Phosphatidylino- sitol-turnovers (CHO-Zellkulturen mit human-$m_1$-Rezeptorsubtyp) erwies sich in vitro die Verbindung (+)-(Propargyloxymethyl)- 1-aza-bicyclo-[2.2.2]octan gegenüber dem Razemat und dem (-)-Enantiomer als stärker wirksam. Die cholinomimetische in-vivo-Wirksamkeit von (+)-(Propargyloxymethyl)-1-aza-bicyclo- [2.2.2]octan wurde am Kaninchen mittels EEG (Thetawellenzunahme) nachgewiesen.

Ebenfalls bekannt sind Quinuclidine der allgemeinen Formel I, worin R als ein über Sauerstoff

verknüpfter heterocyclischer Ring definiert ist. Die britische Patentanmeldung 2 208 385 beschreibt diese Verbindungen jedoch als 5-HT-Antagonisten. Aus der europäischen Patentanmeldung 257 741 sind substituierte 1-Azabicyclo[3.3.1]-nonane der allgemeinen Formel I bekannt, worin R eine über Sauerstoff verknüpfte $C_1$-$C_3$-Alkylgruppe bedeutet.

Die Verbindungen der allgemeinen Formel I können, sofern R ein aliphatischer Rest bedeutet, in Analogie zu L. Stotter et al. in Heterocycles, Vol. 25 (1987), Seite 25 beschriebenen Verfahren hergestellt werden. Ausgehend von den entsprechenden - gegebenenfalls N-geschützten (d.h. das Stickstoffatom des Bicyclus ist durch eine Schutzgruppe Z, z.B. $BH_3$, blockiert) Derivaten (R = H) der allgemeinen Formel I erhält man die erfindungsgemäßen Verbindungen durch Deprotonierung mit starken Basen und anschließende Umsetzung mit einem Alkylierungsreagenz der allgemeinen Formel Y-R, worin R wie zuvor definiert ist und Y eine leicht abspaltbare Austrittgruppe - wie z.B. Halogen, p-Toluolsulfonat u.a. - bedeutet. Die Reaktion wird in polaren inerten organischen Lösungsmitteln, wie z.B. Dimethylformamid, Tetrahydrofuran, Dioxan u.a., durchgeführt.

Während die Deprotonierung und Überführung der Hydroxyverbindung in ein Metallsalz, bevorzugt bei Raumtemperatur oder leicht erhöhter Temperatur durchgeführt wird, erfolgt die anschließende Alkylierung bevorzugt unter Eiskühlung.

Nach erfolgter Umsetzung wird die Schutzgruppe abgespalten und die Verbindungen gegebenenfalls in ihre Säureadditionssalze oder Quartär-Verbindungen überführt, die Reaktionsbedingungen hierzu sind bekannt, bevorzugte Quartärverbindungen sind die Methojodide und Methobromide.

Bevorzugte Reagenzien zur Deprotonierung sind Natriumhydrid, Natriumamid, Alkalialkoholate, wie z.B. Kalium-tert.-Butylat.

Bevorzugte Säureadditionssalze sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Die zur Synthese notwendigen Ausgangsverbindungen, die entsprechenden Hydroxyazabicycloalkane, sind aus dem Stand der Technik bekannt,

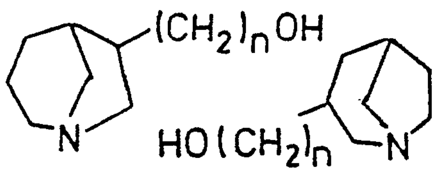

IIa IIb

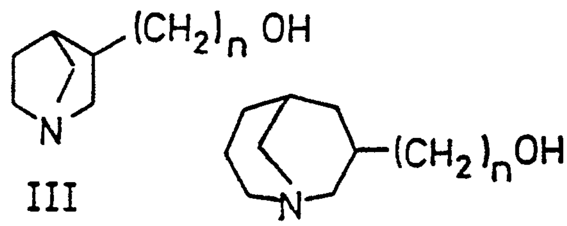

III IV

so z.B. für n = O aus JACS 74, 2215 (1952) (IIa), J. Org. Chem. 33, 4376 (1968) (IIb), der europäischen Patentanmeldung 307 140 (III) und J. Pharm. Science 52, 331 (1954) (IV). Ausgangsverbindungen (R = H), in denen n = 1 oder 2 bedeutet, sind durch Reduktion der entsprechenden Alkylester (X -R = COOAlkyl) erhältlich. Ausgehend von den entsprechenden Ketonen lassen sich die Thiole der allgemeinen Formeln II und III in Analogie zu dem in J. Chem. 43, 1965 beschriebenen Verfahren herstellen.

Ist R in der allgemeinen Formel I ein aromatischer oder heteroaromatischer Rest, so können die Verbindungen der allgemeinen Formel I unter Anwendung der Mitsunobu Reaktion (O. Mitusunobu in Synthesis, 1, 1981, Georg Thieme Verlag, Stuttgart; J. C. S. Perkin I, Seite 462, 1975) durch Umsetzung der entsprechenden hydroxysubstituierten Reaktanden (Formel I R = H, Reagenz HOR, R = Phenyl,Heterocyclus) in Gegenwart von Azodicarbonsäurediethylester und Triphenylphospin erhalten werden. Die Umsetzung erfolgt im allgemeinen in inerten organischen Lösungsmitteln bei Raumtemperatur.

Die Verbindungen der allgemeinen Formel I besitzen wertvolle pharmakologische Eigenschaften. So zeigen die Verbindungen in Bindungsstudien Affinitäten zu muskarinen Rezeptoren und muskarin-agonistische GTP-Shifts (GTP = Guanosintriphosphat). (Birdsall, N.I.M., E.C. Hulme and I.M. Stockton 1984 in T.I.P.S. Supplement, Proc. Internat. Symposium on Subtypes of Muscarinic Receptors, Ed. Hirschowitz, Hammer, Giacchetti, Klirns, Levine; Elsevier p. 4 - 8).

Die Rezeptorbindungsstudien wurden gemäß dem nachstehenden Literaturzitat ausgeführt [A. Closse, H. Bittiger, D. Langenegger und A. Wahner; Naunyn-Schmiedeberg's Arch. Pharmacol. 335, 372 - 377

(1987)].

Tabelle A: Rezeptorbindungsstudien

Radioligand: L(+)cis-[2-Methyl-$^{3}H$]-N,N,N-trimethyl-1, 3-dioxolan-4-methanammonium-jodid NET-647, Fa. NEN (New England Nuclear DU PONT).
Organ: Cerebraler Cortex (Ratte)

**Tabelle A:**

| Beispiel | R* | Ki [nMol/l] |
|---|---|---|
| 1 | -O- 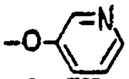 | 120 |
| 4 | $-O-CH_2$-Phenyl | 229 |
| 9 | $-O-C_6H_5$ | 89 |

Als muskarine Agonisten (Cholinomimetika) sind die Substanzen zur Therapie von Krankheiten bei einer Unterfunktion des cholinergen Systems geeignet.

Aufgrund pharmakologischer Befunde sind die Verbindungen z.B. zur Behandlung nachfolgend genannter Krankheiten geeignet: Morbus Alzheimer, senile Demenz, kognitive Störungen und außerdem können die Verbindungen zur Verbesserung der Gedächtnisleistung eingesetzt werden.

Quartäre Verbindungen der allgemeinen Formel I eignen sich besonders zur peripheren Anwendung, so z.B. zur Glaukombehandlung.

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen, z.B. Cerebroatkivatoren und/oder einen peripheren cholinergen Blocker, zur Anwendung gelangen. Geeignete Anwendungsformen sind beispielsweise Tabletten, Kapseln, Zäpfchen, Lösungen, Säfte, Emulsionen oder dispersible Pulver.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die therapeutisch wirksame Einzeldosis liegt im Bereich zwischen 1 und 100 mg.

Die nachfolgenden Beispiele illustrieren die vorliegende Erfindung, ohne sie jedoch in ihrem Umfang zu beschränken:

Pharmazeutische Formulierungsbeispiele

| A) Tabletten | pro Tablette |
|---|---|
| Wirkstoff | 80 mg |
| Milchzucker | 140 mg |
| Maisstärke | 240 mg |
| Polyvinylpyrrolidon | 15 mg |
| Magnesiumstearat | 5 mg |
| | 480 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 60 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 380 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxy-methylstärke und das Magnesiumstearat, vermischt und verpreßt das Gemisch zu Tabletten geeigneter Größe.

C) Ampullen

| | | |
|---|---|---|
| Wirkstoff | | 20 mg |
| Natriumchlorid | | 10 mg |
| bidestilliertes Wasser | q.s. ad | 1,0 ml |

Herstellung:

Der Wirkstoff und das Natriumchlorid werden in bidestilliertem Wasser gelöst und die Lösung in Ampullen steril abgefüllt.

D) Tropfen

| | | |
|---|---|---|
| Wirkstoff | | 5,0 g |
| p-Hydroxybenzoesäuremethylester | | 0,1 g |
| p-Hydroxybenzoesäurepropylester | | 0,1 g |
| entmineralisiertes Wasser | q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

Beispiel 1:

3-Propargyloxy-1-azabicyclo[2.2.1]heptan

5.6 g (0.05 Mol) 1 Azabicyclo[2.2.1]heptan-3-ol werden unter Stickstoffatmosphäre in 150 ml absolutem Tetrahydrofuran gelöst und bei 0° C mit 50 ml 1M Boran-THF-Komplex versetzt. Nach beendeter Zugabe rührt man eine Stunde bei Raumtemperatur, engt zur Trockne ein, nimmt den Rückstand in gesättigter Kochsalzlösung auf und extrahiert mit Dichlormethan. Die vereinigten organischen Phasen werden getrocknet und eingeengt, der Rückstand in 120 ml absolutem THF gelöst und unter Stickstoffatomosphäre portionsweise mit 2.08 g (0.052 Mol) Natriumhydrid versetzt. Nach einer Stunde wird der Ansatz auf 0° C gekühlt, und bei dieser Temperatur werden 17.55 g Propargylbromid als 50 %ige Lösung in THF zugetropft. Es wird 12 Stunden bei Raumtemperatur gerührt, dann zersetzt man überschüssiges Hydrid mit Ethanol, engt ein, nimmt den Rückstand in gesättigter Kochsalzlösung auf und extrahiert mit Dichlormethan. Nach Trocknen und Einengen der vereinigten organischen Phasen erhält man ein Öl, das im Hochvakuum destilliert wird ($Kp_{1\ mbar}$ = 45-46° C). Mit einem Äquivalent Fumarsäure wird das Fumarat hergestellt, aus Ethanol/Ether umkristallisiert und i.V. getrocknet.Man erhält 2.1 g farblose Kristalle der Titelverbindung vom Fp 121-123° C.

$^1$H-NMR(250 MHz, $CD_3OD$, TMS):$\delta$ =6.68 (2H, s, Fumarsäure); 4.47 (1H, m, H-3); 4.21 (2H, m, $CH_2$-8); 3.73-2.86 (7H, m, $CH_2$-2, 6, 7; H-4); 2.95 (1H, t, J=3Hz, H-9); 2.30; 1.96 (2H, m, $CH_2$-5).

Beispiel 2:

3-Phenoxy-1-azabicyclo[2.2.2]octan

3.82 g (0.03 Mol) 3-Hydroxychinuclidin, 2.82 g (0,03 Mol) Phenol und 7,96 g (0.03 Mol) Triphenylphosphin und 5.22 g (0.03 Mol) Azodicarbonsäurediethylester werden in 150 ml absolutem THF gelöst und 2 Tage bei Raumtemperatur gerührt. Man engt zur Trockne ein, nimmt den Rückstand in 20 ml 6N HCl und 50 ml $H_2O$ auf und extrahiert mit Ether. Die Wasserphase wird alkalisch gestellt und mit Ethylacetat ausgeschüttelt, die vereinigten Essigester-Phasen werden getrocknet und eingeengt. Nach einer Destillation erhält man 3.6 g farbloses Öl ($Kp_{0.1\ mbar}$ = 104-105° C).

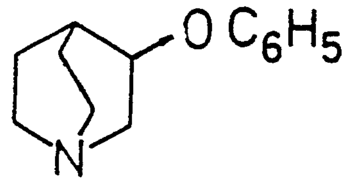

$^1$H-NMR (250 MHz, $CDCl_3$, TMS(:δ=7.26; 6.87 (5H, m, aryl-H); 4.36 (1H, m, H-3); 3.34-2.63 (6H, m, $CH_2$-2, 6, 7); 2.19-1.27 (5H, m, CH-4; $CH_2$ 5, 8).

Die Base wird in ethanolischer Lösung in das Fumarat übergeführt, das mit Ether gefällt und aus Acetonitril umkristallisiert wird.

Man erhält 4.1 g farblose Kristalle vom Fp. 122-124° C.

Beispiel 3:

(+)- und (-)-3-(Propargyloxymethyl)-1-azabicyclo[2.2.2]octan

±-Essigsäure-(3-chinuclidinylmethyl)-ester.

Racemisches 3-Chinuclidinylmethanol wird nach literaturbekannten Verfahren, z.B. mittels Cyanhydrinsynthese aus Chinuclidin-3-on nach Helv. Chim. Acta 37, 1695 (1954) hergestellt und mit Acetylchlorid und Triethylamin in Chloroform als Solvens bei Raumtemperatur acyliert, wobei der racemische Essigsäure-(3-chinuclidinylmethyl)ester in 82 % Ausbeute und einem Siedepunkt von 130 - 134° bei 30 mbar anfällt.

(-)-3-Chinuclidinylmethanol

26,5 g ±-Essigsäure-(3-chinuclidinylmethyl)-ester und 21.7 g L-(+)-Weinsäure werden in 275 ml 95-prozentigem Ethanol zum Sieden erhitzt; bei der anschließenden langsamen Abkühlung erhält man kristallines Weinsäuresalz, das aus 95-prozentigem Ethanol ca. 5 x bis zur Drehwinkelkonstanz umkristallisiert wird. Die so erhaltenen 13.5 g Salz vom Drehwert $[\alpha]_D^{20}$ = -16.93° (c = 2,$H_2O$) werden in 80 ml 2 N Natronlauge zwei Stunden bei Raumtemperatur gerührt, die Lösung anschließend mit 80 g Pottasche versetzt und der Ansatz extraktiv mit Chloroform aufgearbeitet. Auf diese Weise setzt man unter gleichzeitiger Esterverseifung 5,6 g (-)-3-Chinuclidinylmethanol als helles Öl mit $[\alpha]_D^{20}$ = -66° (c = 1, 2, in 1 N HCl) frei.

Die analytische Überprüfung der Enantiomerenreinheit erfolgte nach Derivatisierung mit Phenylisocyanat mittels HPLC an einer Chiralcel-OD-Säule und ergab eine Enantiomerenverteilung von 98,6 % (-) : 1,4 % (+)-Enantiomer.

3-Chinuclidinylmethanol

Die gesammelten Mutterlaugen, die bei der Herstellung des (-)-3-Chinuclidinylmethanols anfallen, werden im Vakuum eingeengt. Den Rückstand nimmt man in wenig Wasser auf, stellt mit Pottasche alkalisch und extrahiert mit Chloroform. Die so erhaltenen 19,5 g des optisch angereicherten Essigsäure-(3-chinuclidinylmethyl)-esters werden mit 16.0 g D-(+)-Weinsäure in 200 ml 95 %igem Ethanol in das Diastereomerensalz überführt. Man erhält nach 5-maligem Umkristallisieren aus 95 %igem Ethanol 12.7 g Salz vom Drehwert $[\alpha]_D^{20}$ = 16.8 (c = 2, $H_2O$), aus dem analog wie beim (-) Enantiomer mittels 75 ml 2 N Natronlauge 4,7 g (+)-3-Chinuclidinylmethanol als helles Öl mit $[\alpha]_D^{20}$ = + 66.5° (c = 1, in 1 N HCl) freigesetzt wird.

Nach Derivatisierung mit Phenylisocyanat wurde die Enantiomerenreinheit mittels HPLC an einer Chiralcel-OD- Säule mit 97,4 % (+)- : 2,6 % (-) Enantiomer bestimmt.

3a: (+)-3-(Propargyloxymethyl)-1-azabicyclo[2.2.2]octan

5,6 g (+)-3-Chinuclidinylmethanolhydrochlorid, 1.32 g Natriumborhydrid und 100 ml Tetrahydrofuran

werden über Nacht gerührt. Vom Filtrat wird das Solvens abgezogen, der Rückstand in Essigester aufgenommen und die erhaltene Lösung mit gesättiger Kochsalzlösung gewaschen. Nach dem Trocknen und Abziehen des Solvens bleiben 3,4 g (+)-3-Chinuclidinylmethanol-Borankomplex als helles Öl zurück.

3.4 g des Borankomplexes werden in 100 ml Tetrahydrofuran 30 Minuten mit 2.63 g 60 %igem Natriumhydrid bei Raumtemperatur gerührt, anschließend mit 4.89 g 80 %igem Propargylbromid umgesetzt und 6 weitere Stunden gerührt. Das Reaktiongemisch wird mit Alkohol vorsichtig zersetzt, das Solvens abgezogen, der Rückstand in 150 ml Essigester aufgenommen, die Lösung mit gesättigter Kochsalzlösung gewaschen und das Solvens erneut abgezogen. Zur Zerstörung der Boranschutzgruppe nimmt man den Rückstand in 50 ml Aceton auf und rührt mit 20 ml 3 N HCl über Nacht. Nach dem Abdampfen des Acetons wird die wäßrige Phase mit Essigester gewaschen, mit Pottasche alkalisch gestellt und mit Essigester extrahiert. Der Extraktionsrückstand wird an Kieselgel mit Essigester : Methanol : $NH_3$ = 85 : 15 : 1 als Eluens flashchromatographiert und ergibt 4,3 g Propargylether, der in Alkohol mit der berechneten Menge Fumarsäure in das Fumarsalz übergeführt wird, das aus Alkohol-Ether umgefällt wird und in 3.9 g Ausbeute mit Fp. 132 - 133° C und $[\alpha]_D^{20}$ = + 28.47° (c = 1, Methanol) anfällt.

3 b: (-)-3-(Propargyloxymethyl)-1-azabicyclo[2.2.2]octan

Analog 3a wird in (-)-3-Chinuclidinylmethanol die Boranschutzgruppe eingeführt, die Verätherung mit Propargylbromid durchgeführt und nach der Abspaltung der Boranschutzgruppe die freie Base in das Fumarat überführt mit Fp. 132 - 133° C und $[\alpha]_D^{20}$ = -28.42° (c = 1, Methanol).

In Analogie zu den allgemein beschriebenen Syntheseverfahren und Beispielen können folgende Verbindungen der allgemeinen Formel I hergestellt werden.

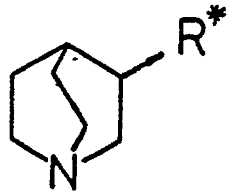

| Bsp. | $R^*$ | Schmp. °C |
|---|---|---|
| 1 | 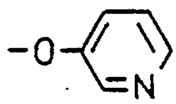 ·2HCl | 236 (Zers.) |
| 2 | 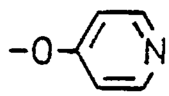 | 164 - 166<br>Fumarat |
| 3 | 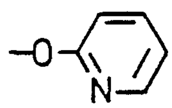 | 160 - 161<br>Fumarat |
| 4 | 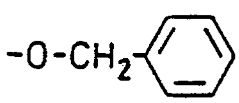 | 135-136<br>Fumarat |
| 4a | (+)Enantiomer 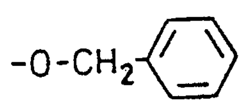 | $[\alpha]_D$ =<br>+ 27,5<br>(c = 1, $H_2O$) |

| Bsp. | R* | Schmp. °C |
|---|---|---|
| 4b | (-)Enantiomer 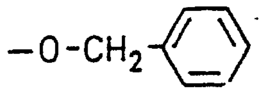 | $[\alpha]_D$ = - 25,6 (c = 1, $H_2O$) |
| 5 | 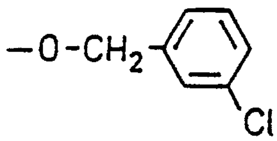 | 110 (Zers.) Fumarat |
| 6 | 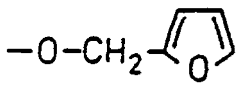 | 110-112 Fumarat |
| 7 | 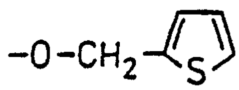 | 112-114 Fumarat |
| 8 | 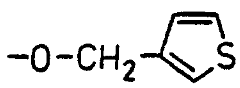 | 108-110 Fumarat |
| 9 | 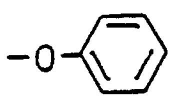 | 122-124 Fumarat |

| Bsp. | R* | Schmp. °C |
|---|---|---|
| 9a | (+)-Enantiomer[Δ] 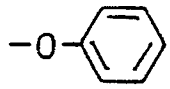 | $[\alpha]_D$ = + 22.2 (c = 2, $H_2O$) |
| 9b | (-)-Enantiomer[Δ] 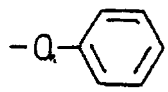 | $[\alpha]_D$ = - 22,9 (c = 2, $H_2O$) |
| 10 | 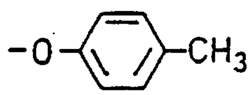 | 147 - 149 Fumarat |
| 10a | 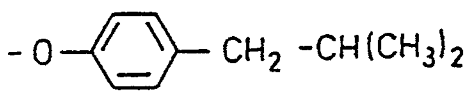 | |
| 10b | 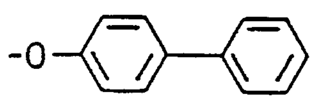 | |
| 10c | 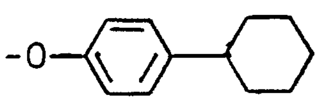 | |
| 11 | 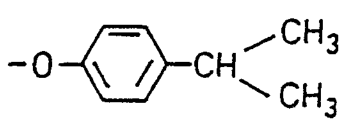 | 126 - 129 Fumarat |

Δ Die Reaktion erfolgt unter Inversion.

| Bsp. | R* | Schmp. °C |
|---|---|---|
| 11a | 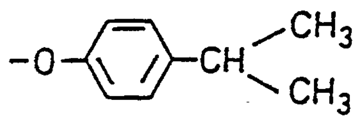 | (+)-Enantiomer |
| 11b | 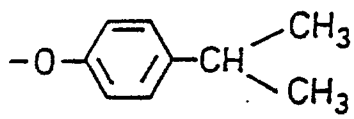 | (-)-Enantiomer |
| 12 | 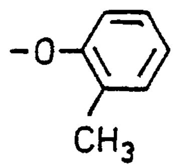 | 125 - 126 Fumarat |
| 13 | 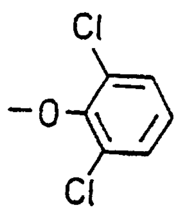 | 147 - 149 Fumarat |
| 14 | 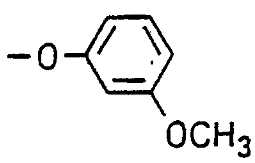 | 128-131 Fumarat |
| 15 | 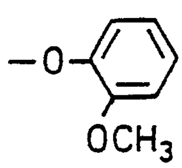 | |
| 16 | 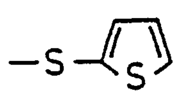 | |

| Bsp. | R* | Schmp. °C |
|---|---|---|
| 17 | 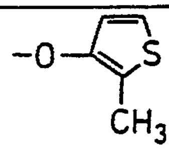 | |
| 18 | 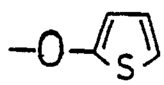 | |
| 19 | 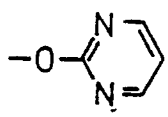 | 197-198 Fumarat |
| 20 | 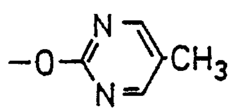 | |
| 21 | 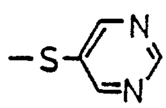 | |
| 22 | 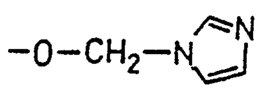 | |
| 23 | 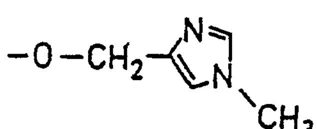 | |
| 24 | 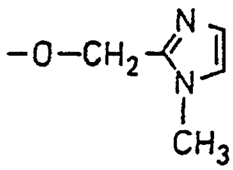 | |
| 25 | 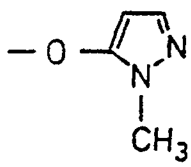 | |

| Bsp. | $R^*$ | Schmp. °C |
|---|---|---|
| 26 | 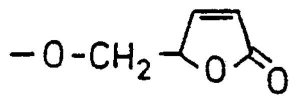 | |
| 27 | 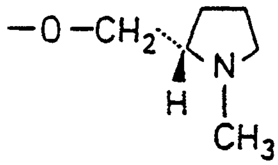 | |
| 28 | 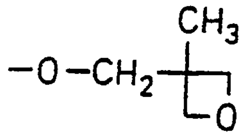 | |
| 29 | 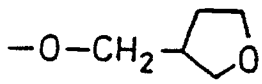 | |
| 30 | 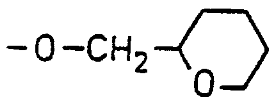 | |
| 31 | 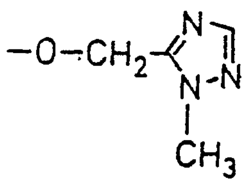 | |
| 32 | 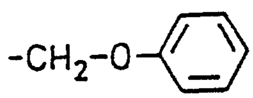 | 117 - 118 Fumarat |
| 33 | 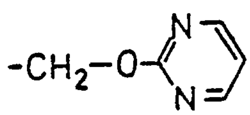 | 189 - 191 Fumarat |

| Bsp. | R* | Schmp. °C |
| --- | --- | --- |
| 33a | 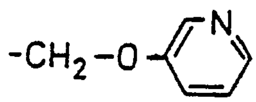 | 158 - 160 Fumarat |
| II. | 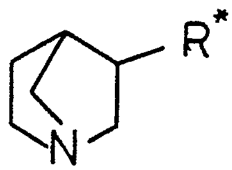 | |
| 34 | $-O-CH_2-C{\equiv}CH$ | 121-127 Fumarat |
| 35 | $-O-CH_2-CH{=}CH_2$ | |
| 36 | 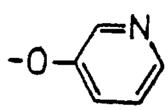 | |
| 37 | 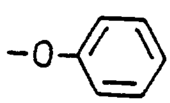 | 114 - 116 Oxalat |

| Bsp. | R* | Schmp. °C |
|---|---|---|
| 38 | 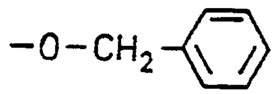 | |
| 39 | 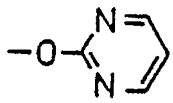 | |
| 40 | 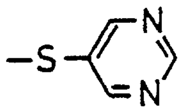 | |
| III. | 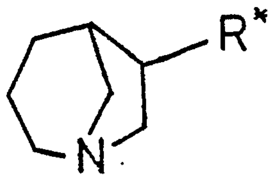 | |
| 41 | endo $-O-CH_2-C{\equiv}CH$ | 121-124<br>Oxalat |
| 42 | exo $-O-CH_2-C{\equiv}CH$ | Öl<br>Oxalat |
| 43 | 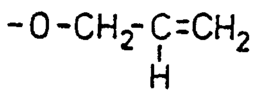 | 93-95<br>Oxalat |
| 44 | 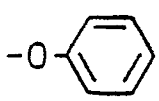 | |
| 45 | 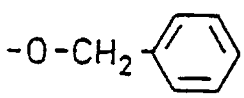 | |

| Bsp. | $R^*$ | Schmp. °C |
|---|---|---|
| 46 | 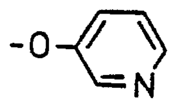 | |
| 47 | 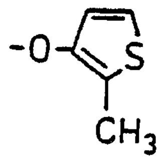 | |
| 48 | 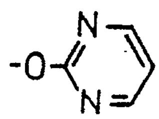 | |
| IV. | 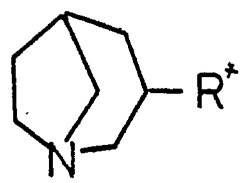 | |
| 49 | endo $-O-CH_2-C{\equiv}CH$ | 148-149 Fumarat |
| 49a | exo $-O-CH_2-C{\equiv}CH$ | 147 - 148 Fumarat |
| 50 | exo $-O-CH_2-CH{=}CH_2$ | 99-101 Fumarat |

| Bsp. | | $R^{*}$ | Schmp. °C |
|---|---|---|---|
| 50a | endo | $-O-CH_2-CH=CH_2$ | 113-114 Fumarat |
| 51 | exo | $-O-CH_2-CH_3$ | 147-148 Fumarat |
| 51a | endo | $O-CH_2-CH_3$ | 115-117 Fumarat |
| 52 | | $-O-C_6H_5$ | |
| 53 | | $-O-CH_2-C_6H_5$ | |
| 54 | | 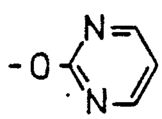 | |
| 55 | | 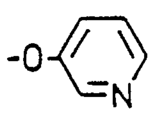 | |

| Bsp. | $R^*$ | Schmp. °C |
|---|---|---|
| V. | 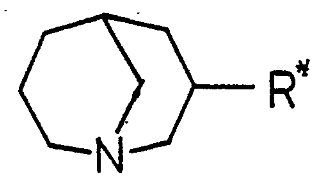 | |
| 56 | $-O-CH_2-CH=CH_2$ | |
| 57 | $-O-CH_2-C\equiv CH$ | |
| 58 | $-O-C_6H_5$ | |
| 59 | 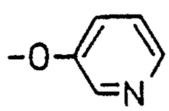 | |
| 60 | 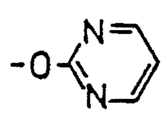 | |
| 61 | $-O-CH_2-C_6H_5$ | |
| 62 | $O-CH_2-CH_3$ | |

**Patentansprüche**

**1.** Neue bicyclische 1-Aza-cycloalkane der allgemeinen Formel

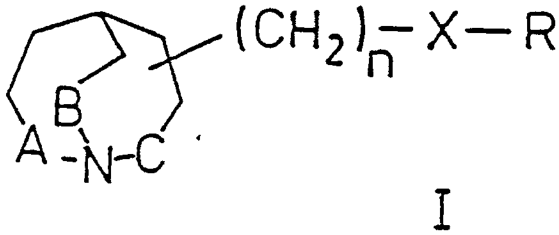

I

worin

| | |
|---|---|
| R | einen Alkylrest mit 1 bis 6 Kohlenstoffatomen, einen Alkenylrest mit 3 bis 6 Kohlenstoffatomen, einen Alkinylrest mit 3 bis 6 Kohlenstoffatomen, wobei der Alkyl-, Alkenyl oder Alkinylrest durch Phenyl, substituiertes Phenyl, gegebenenfalls substituiertes Biphenyl, ein gegebenenfalls substituiertes Oxetan oder einen gegebenenfalls substituierten 5-, 6- oder 7-gliedrigen Heterocyclus substituiert sein kann, oder |
| R | ein gegebenenfalls substituiertes Phenyl, ein gegebenenfalls substituiertes Biphenyl, einen gegebenenfalls substituierten 5-, 6- oder 7-gliedrigen Heterocyclus; |
| X | Sauerstoff oder Schwefel, |
| A, B und C | unabhängig $CH_2$ oder eine Einfachbindung und |
| n | = 0, 1 oder 2 |

bedeuten können, gegebenenfalls in Form ihrer Racemate, ihrer Enantiomere, ihrer Diasterecmere und ihrer Gemische sowie gegebenenfalls ihre pharmakologisch unbedenkliche Säureadditionssalze wie auch ihre Quartärsalze.

2. Neue bicyclische 1-Azacycloalkane der allgemeinen Formel I gemäß Anspruch 1 worin X Sauerstoff, n = 0 oder 1 und der Substituent $(CH_2)_n$-X-R in $\alpha$ oder $\beta$-Position zum carbocyclischen Brückenkopf steht, und R eine Alkylgruppe mit 1 bis 3 Kohlenstoffatomen, eine Alkenylgruppe mit 3 oder 4 Kolenstoffatomen, eine Alkinylgruppe mit 3 oder 4 Kohlenstoffatomen, eine gegebenenfalls substituierte Phenylgruppe, einen gegebenenfalls substituierten 5- oder 6-gliedrigen aromatischen Heterocyclus bedeuten können, gegebenenfalls in Form ihrer Racemate, ihrer Enantiomere, ihrer Diastereomere und ihrer Gemische sowie gegebenenfalls ihre pharmakologisch unbedenklichen Säureadditionssalze wie auch ihre Quartärsalze.

3. Neue bicyclische 1-Azacycloalkane gemäß Anspruch 1 der allgemeinen Formel

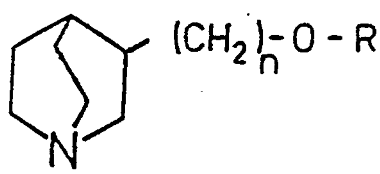

worin

n = 0 oder 1;

und

R einen Rest der Formel

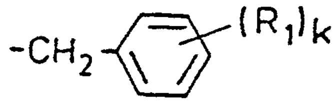

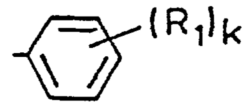

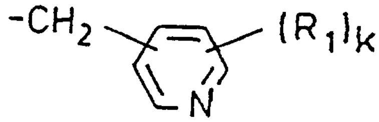

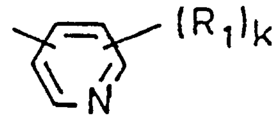

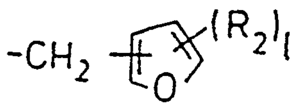

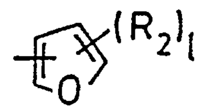

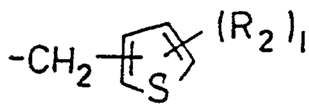

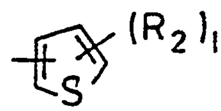

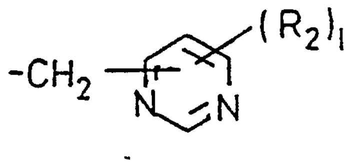
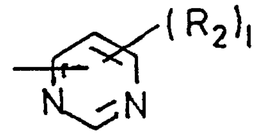

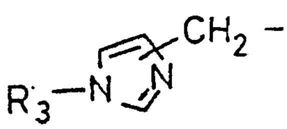
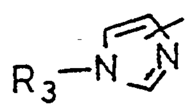

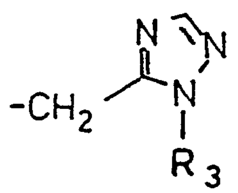
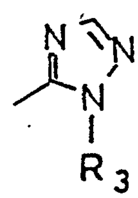

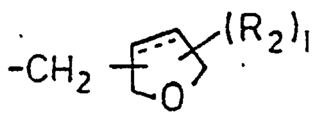
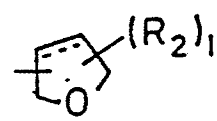

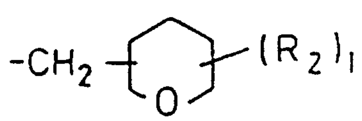
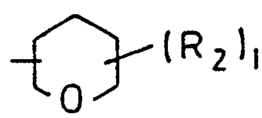

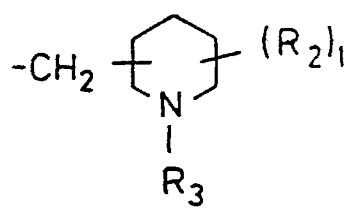
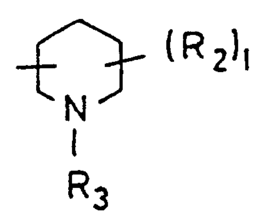

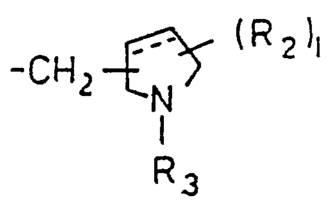
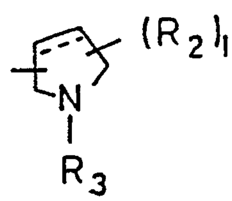

worin

$R_1$ Wasserstoff, $C_1$ - $C_4$ Alkyl, bevorzugt Methyl, $C_1$ - $C_4$-Alkoxy, bevorzugt Methoxy, Amino, $c_1$ - $C_4$-Alkylamino, $C_1$ - $C_4$-Dialkylamino, Halogen, Hydroxy, $C_3$ - $C_6$-Cycloalkyl, gegebenenfalls substituiertes Phenyl, = 0;

k 1, 2 oder 3, wobei k > 1 alle $R_1$ gleich oder verschieden sein können,

$R_2$ Wasserstoff, $C_1$ - $C_4$-Alkyl, bevorzugt Methyl, $C_1$-$C_4$-Alkoxy, bevorzugt Methoxy, Halogen, = 0;

l 1 oder 2, bevorzugt 1, wobei bei l = 2 $R_2$ gleich oder verschieden sein kann;

$R_3$ Wasserstoff, $C_1$-$C_4$-Alkyl, bevorzugt Methyl bedeuten können,

oder der allgemeinen Formel

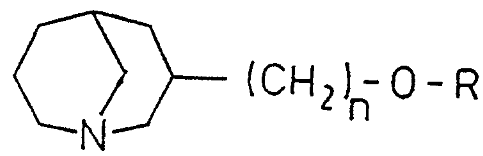

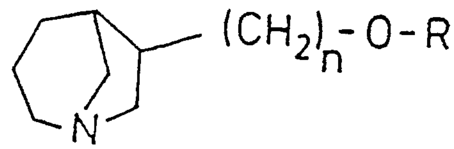

oder

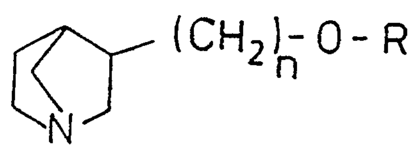

worin

n = null oder 1, bevorzugt null;

R = $C_3$-Alkinyl, $C_3$-Alkenyl, einen Rest der allgemeinen Formel

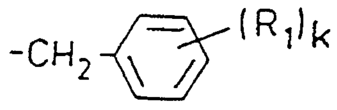
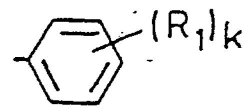

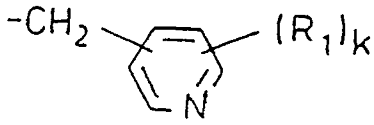
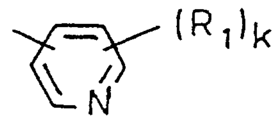

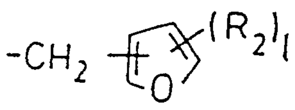
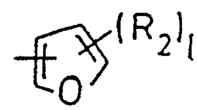

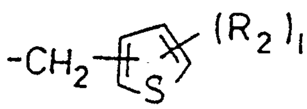
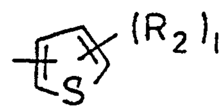

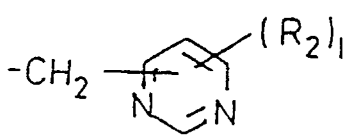
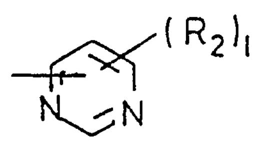

worin $R_1$, $R_2$, k und l wie zuvor definiert sind; oder der allgemeinen Formel

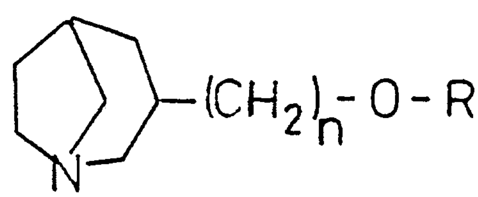

worin
n 0 null oder 1;
R = $C_3$-Alkinyl, $C_3$-Alkenyl, Methyl, Ethyl, Propyl,

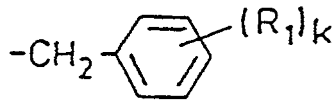

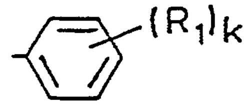

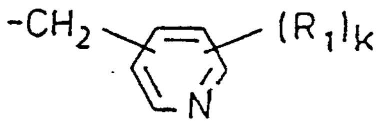

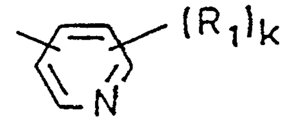

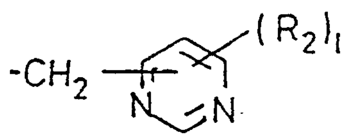

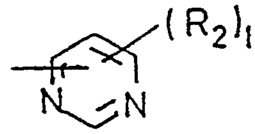

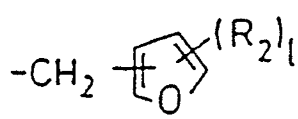

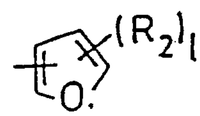

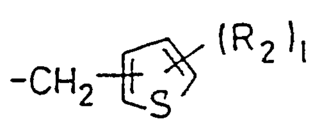

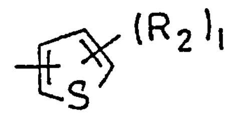

worin $R_1$, $R_2$, k und l wie zuvor definiert sind, in Form ihrer Racemate, ihrer Enantiomere, ihrer Diastereomere und ihrer Gemische sowie ihre pharmakologisch unbedenklichen Säureadditionssalze wie auch ihre Quartärsalze.

4. Verwendung von Quinuclidinen der allgemeinen Formel I gemäß Anspruch 1, 2 oder 3 gegebenenfalls in Form ihrer Racemate, ihrer Enantiomere, ihrer Diastereomere und ihrer Gemische sowie ihrer pharmakologisch unbedenklichen Säureadditionssalze wie auch ihrer Quartärsalze als Arzneimittel.

5. Pharmazeutische Zubereitung enthaltend eine Verbindung der allgemeinen Formel I gemäß Anspruch 1, 2 oder 3 sowie übliche Hilfs- und/oder Trägerstoffe.

6. Verfahren zur Herstellung von pharmazeutischen Zubereitungen gemäß Anspruch 5, dadurch gekennzeichnet, daß man Verbindungen der allgemeinen Formel I mit üblichen galenischen Hilfs- und/oder Trägerstoffen mischt.

7. Verfahren zur Herstellung von Verbindung der allgemeinen Formel 1, gemäß Anspruch 1, 2 oder 3 dadurch gekennzeichnet, daß man eine Verbindung der allgemeinen Formel

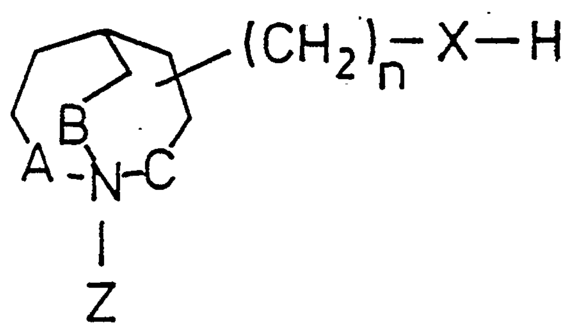

worin n und x wie zuvor definiert sind und Z eine Schutzgruppe bedeutet (im Fall von X = S, kann auf die Schutzgruppe Z verzichtet werden), deproniert und mit einem Alkylierungsreagenz der Formel

Y - R

worin R wie zuvor definiert ist und Y eine leicht abspaltbare Gruppe bedeutet umsetzt, anschließend die Schutzgruppe abspaltet und gegebenenfalls in ihrer Säureadditionssalze oder Quartärsalze überführt und/oder gegebenenfalls in ihre optisch aktiven Verbindungen trennt.

**8.** Verfahren zur Herstellung von Verbindungen der allgemeinen Formel I, gemäß Anspruch 1, 2 oder 3, worin R gleich gegebenenfalls substituiertes Phenyl, gegebenenfalls substituiertes Biphenyl, einen gegebenenfalls substituierten 5-, 6- oder 7-gliedrigen Heterocyclus bedeutet, dadurch gekennzeichnet, daß man eine Verbindung der Formel

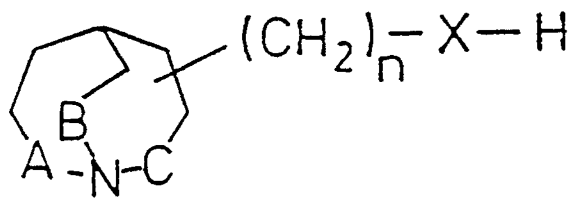

mit einer Verbindung der Formel HOR (R = Phenyl, Heterocyclus) in Gegenwart von Triphenylphosphin und Azodicarbonsäurealkylester umsetzt.

**9.** (+)-(Propargyloxymethyl)-1-aza-bicyclo[2.2.2]octan, dessen Säureadditionssalze sowie dessen quaternäre Salze.

**10.** Verwendung einer Verbindung der allgemeinen Formel I gemäß einem der Ansprüche 1, 2, 3 oder 9 zur Herstellung eines Arzneimittels zur Behandlung von Zuständen oder Krankheiten, die auf einer Unterfunktion des cholinergen Systems beruhen.

**11.** Verwendung einer Verbindung der allgemeinen Formel I gemäß einem der Ansprüche 1,2, 3 oder 9 zur Herstellung eines Arzneimittels zur Behandlung von Alzheimer, seniler Demenz oder zur Verbesserung der Gedächtnisleistung.

**12.** Verwendung von Verbindungen der allgemeinen Formel I gemäß einem der Ansprüche 1,2, 3 oder 9 zur Herstellung eines Arzneimittels mit 5-HT-antagonistischen Eigenschaften.

Europäisches Patentamt

# EUROPÄISCHER RECHERCHENBERICHT

Nummer der Anmeldung

EP 91108054.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
| X | AT - B - 291 273 (SOCIETE GENERALE DE RECHERCHES ET D'APPLICATIONS SCIENTIFIQUES SOGERAS IN PARIS) * Anspruch 1; Seite 13, Zeile 46 - Seite 22, Zeile 27 * -- | 1,4-6, 10,12 | C 07 D 453/02<br>C 07 D 471/08<br>C 07 D 487/08<br>A 61 K 31/435<br>A 61 K 31/55<br>A 61 K 31/395 |
| X | AT - B - 298 502 (SOCIETE GENERALE DE RECHERCHES ET D'APPLICATIONS SCIENTIFIQUES SOGERAS IN PARIS) * Anspruch 1; Seite 2, Zeile 38 - Seite 6, Zeile 24 * -- | 1,4-6, 10,12 | |
| X | DE - A - 1 800 823 (AKTIEBOLAGET ASTRA) * Ansprüche 1,4,7-9,12; Tabellen I,II * -- | 1,4-6, 10 | |
| D,X | GB - A - 2 208 385 (JOHN WYETH & BROTHER LIMITED) * Ansprüche 1-3,9,11; Beispiel 4 * -- | 1-6 | RECHERCHIERTE SACHGEBIETE (Int. Cl.⁵)<br>C 07 D 453/00<br>C 07 D 471/00<br>C 07 D 487/00<br>A 61 K 31/00 |
| D,X | EP - A2 - 0 257 741 (BEECHAM GROUP PLC) * Ansprüche 1-5,9-12 * -- | 1,2, 4-6, 10,11 | |
| X | HELVETICA CHIMICA ACTA, Band 57, 1974 W. ECKHARDT, C.A. GROB "Synthese und Basizität 4-heterosubstituierter Chinuclidine" Seiten 2339-2345 * Seiten 2339,2341,2342 * -- | 1,7 | |
| X | HETEROCYCLES, Band 25, 1987 | 1,7 | |

Der vorliegende Recherchenbericht wurde für alle Patentansprüche erstellt.

| Recherchenort | Abschlußdatum der Recherche | Prüfer |
|---|---|---|
| WIEN | 23-08-1991 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-
EP 91108054.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| | P.L. STOTTER et al. "Quinuclidine-boranes as intermediates in formation and isolation of functionalized quinuclidine systems" Seiten 251-258 * Seite 253 * -- | | |
| P,D, X | EP - A1 - 0 370 415 (BOEHRINGER INGELHEIM KG) * Zusammenfassung; Ansprüche 1-6; Seite 3, Zeile 12 - Seite 5, Zeile 34; Beispiele * ---- | 1,2, 4-7, 10,11 | |

RECHERCHIERTE SACHGEBIETE (Int. Cl.⁵)

Der vorliegende Recherchenbericht wurde für alle Patentansprüche erstellt.

| Recherchenort | Abschlußdatum der Recherche | Prüfer |
|---|---|---|
| WIEN | 23-08-1991 | MAZZUCCO |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument